# EUROPEAN PATENT SPECIFICATION

(11) **EP 3 773 856 B1**
(45) Date of publication and mention of the grant of the patent: **09.07.2025**
(21) Application number: 19784337.8
(22) Date of filing: 09.04.2019
(51) Int. Cl.: A61M 25/06

(54) **IV CATHETER WITH A TIP PROTECTOR**
IV-KATHETER MIT EINEM SPITZENSCHUTZ
CATHÉTER INTRAVEINEUX DOTÉ D'UN PROTECTEUR DE POINTE

(30) Priority: 11.04.2018 US 201862656200 P
(43) Date of publication of application: 17.02.2021
(73) Proprietor: ICU Medical, Inc., San Clemente, CA 92673 (US)
(72) Inventor: KRAUSE, Eric Jason, Plymouth, Minnesota 55442 (US); JOHNSON, Daniel Casey, Plymouth, Minnesota 55442 (US); GORAL, David J., Plymouth, Minnesota 55442 (US); BREINDEL, Jay T., Plymouth, Minnesota 55442 (US)
(74) Representative: Harris, Oliver John Richard
(86) International application number: PCT/US2019/026469
(87) International publication number: WO 2019/199736

(56) References cited:
- WO-A1-2017/029374
- WO-A1-2017/214110
- US-A1- 2005 182 363
- US-A1- 2010 191 189
- US-A1- 2013 317 426
- US-A1- 2015 051 584
- US-A1- 2016 135 841

## Description

### TECHNICAL FIELD

The present disclosure relates generally to safety catheters, and more particularly to a safety catheter having a passive release mechanism configured to shield a sharp distal tip of a needle cannula prior to release of a catheter hub from a catheter insertion device.

### BACKGROUND

Intravenous (IV) therapy is a versatile technique used for the administration of medical fluids to and withdrawal of bodily fluids from patients. IV therapy has been used for various purposes, such as the maintenance of blood and electrolyte balance, the transfusion of blood, the administration of nutritional supplements, chemotherapy, and the administration of drugs and medications. These fluids, collectively referred to herein as medicaments, may be administered intravenously by injection through a hypodermic needle, or intermittently or continuously by infusion using a needle or catheter. A common intravenous access device utilized by clinicians is the Peripheral Intravenous Catheter (PIVC).

A PIVC is made of a soft, flexible plastic or silicone, generally between fourteen to twenty-four gauge in size. In the conventional venipuncture procedure, a catheter is inserted into a vein in the patient's hand, foot, or the inner aspect of the arm or any vein in the body that will accept an IV catheter. In order to place the IV catheter into the patient's vein, a sharp introducer needle is used to puncture the skin, tissue, and vein wall to provide a path for placement of the catheter into the vein.

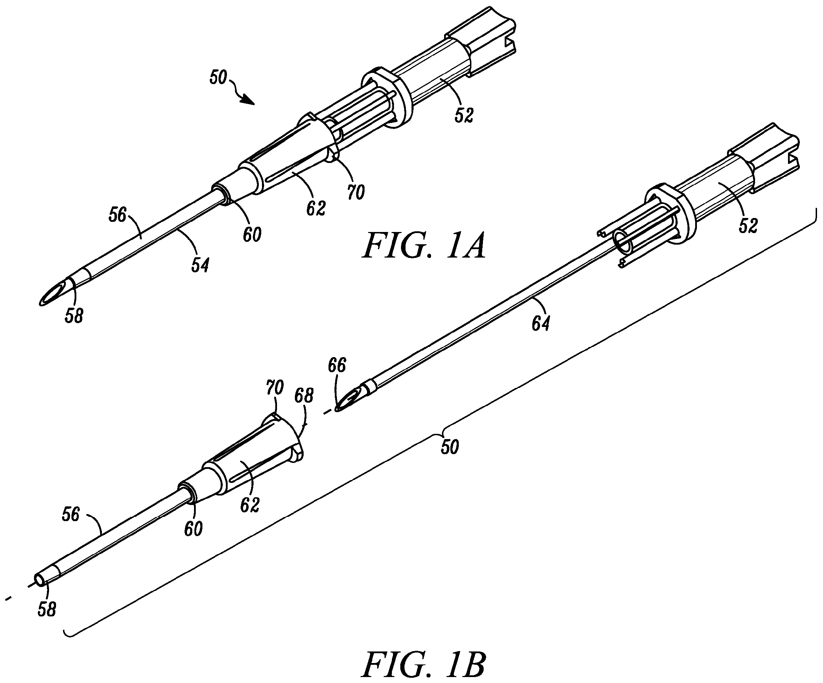

Referring to FIGS. 1A-B, a conventional catheter insertion assembly 50 including a catheter insertion device 52 configured to insert an "over the needle" catheter 54 is depicted. Catheter 54 generally includes a catheter tube 56 having a distal end 58 for insertion into a biological site, a proximal end 60 and a flexible wall defining a lumen extending therebetween. Frequently, the proximal end 60 of the catheter tube 56 is operably coupled to a catheter hub 62. Catheter 54 can be operably coupleable to the catheter insertion device 52, in part by positioning the catheter 54 coaxially over a needle cannula 64 of the catheter insertion device 52. The catheter 54 thus rides with the needle cannula 64 through the skin, tissue and vein wall into the patient's vein.

Various catheter insertion devices have been developed to provide a needle for catheterization. One such example of this type of catheter insertion device is marketed by Smiths Medical ASD, Inc. of St. Paul, Minn., under the JELCO and INTUITIV trademarks, and are described in U.S. Pat. No. 8,257,322 and U.S. Pat. Publ. Nos. 2011/0319838; and 2017/0095617.

In other cases, the catheter insertion device provides a safety needle assembly that functions to house the sharpened tip of the needle to reduce the likelihood of an inadvertent needle stick. Examples of this type of catheter insertion device are marketed by Smiths Medical ASD, Inc. under the PROTECTIV and VIAVALVE trademarks, and are described in U.S. Pat. Nos. 5,000,740; 7,736,342 and U.S. Pat. Publ. No. 2016/0220791.

Once the catheter tube 56 has been entered into the patient's vein, the needle cannula 64 and catheter 54 are lowered towards the skin of the patient to decrease the entry angle, and the catheter tube 56 is advanced slightly into the vein. The connection between the catheter 54 and the needle cannula 64 is then loosened, so that the catheter tube 56 can be advanced further into the vein as desired, and the needle cannula 64 can be withdrawn from the catheter 54.

In some cases, the catheter 54 can include a tip protector through which at least a portion of the needle cannula 64 passes. The tip protector can be configured to enclose or otherwise shield a sharp distal tip 66 of the needle cannula 64 after it has been withdrawn from the catheter tube 56. One form of tip protector involves a clip that fits within the catheter hub 62. Such clips can include thin webs of metal or the like which are bent or otherwise formed to have a back wall and one or more distally extending walls, all generally of the same thickness. In a ready state of the clip, the shaft of the needle cannula 64 passes through an aperture in the back wall of the clip and against a pair of distally extending arms, to pass into the catheter tube 56. The needle cannula 62 can be pulled proximally relative to the clip, so as to bring the distal tip 66 within the clip proximal to the back wall, whereupon the arms are configured to close to block distal reemergence of the distal tip 66. Further proximal movement of the needle cannula 64 pulls the tip protector out of the catheter hub 62.

The catheter hub 62 can be coupled to an administration set or syringe for introducing fluids into, and/or withdrawing bodily fluid from, the patient. The catheter hub 62 can have an open proximal end 68 adapted to receive a male luer taper into a cavity defined therein to establish a fluid connection between the patient's vasculature and a luer taper. The proximal end 68 can also be provided with external ears 70, or the like, to secure the luer taper in the catheter hub 62, such as when the luer taper is coupled with a male luer lock collar of an administration set or syringe.

Under normal conditions, after withdrawal of the needle cannula 64 and before a luer taper is inserted into the catheter hub 62, blood flows through the catheter tube 54 and into the interior cavity of the catheter hub 62. To limit blood flow, the catheter hub 62 can include a valve or septum that seals the needle cannula path after the needle cannula 64 has been withdrawn from the catheter 54, thereby inhibiting blood or bodily fluid from the patient from escaping from the catheter 54 to the surrounding environment.

Catheter insertion devices 50 which attempt to lock the catheter 54 to the catheter insertion device 52 during insertion have been created. However, such devices could be improved to consistently enable a smooth release of the catheter hub 62 from the needle cannula 64, particularly in a way that reduces or eliminates the risk of an inadvertent needle stick. Accordingly, Applicants of the present disclosure have identified a need for a safety catheter insertion assembly that includes a mechanism for smoothly and passively releasing the catheter from the catheter insertion device upon retraction of the needle cannula. US 2010/191189 A1 relates to a catheter and introducer needle assembly that includes a needle shield that will safely shield the sharp distal tip of the introducer needle after the needle has been used to insert the catheter into a patient.

### SUMMARY OF THE DISCLOSURE

The invention is defined in claim 1.

Embodiments of the present disclosure provide a safety catheter insertion assembly that includes a mechanism for smoothly and passively releasing a catheter from a catheter insertion device upon withdrawal of a needle cannula into a safety clip, thereby inhibiting the risk of an inadvertent needle stick, while improving the catheter insertion process.

One embodiment of the present disclosure provides a passive safety catheter assembly including a catheter hub and catheter tube extending distally thereof, and a catheter insertion device. The catheter insertion device including a needle cannula, handle, and safety clip assembly. The needle cannula having a proximal portion, an elongate body and sharp distal tip. The handle operably coupled to the proximal portion of the needle cannula. The safety clip assembly including a housing and safety clip. The safety clip including a first and second guard arm, each guard arm including a respective first and second retention tab defining a respective first and second aperture configured to enable passage of the elongate body and sharp distal tip of the needle cannula therethrough and a respective first and second hook feature configured to enable the safety clip to selectively grip the catheter hub. The safety clip further including one or more safety clip retention tabs configured to secure the safety clip at least partially within the housing. Wherein the safety clip assembly is configured to transition between a first position in which the first and second guard arms are restrained against their natural bias by the passage of the needle cannula through an alignment of the first and second apertures of the first and second retention tabs, such that the first and second hook features are in gripping contact with the catheter hub, and a second position in which the needle cannula is proximally withdrawn through the first and second apertures to enable the first and second guard arms to be naturally biased apart, such that the first and second apertures of the retention tabs become unaligned to trap the sharp distal tip of the needle cannula within the safety clip, and the first and second hook features release gripping contact with the catheter hub.

In one embodiment, the safety clip can be constructed of a resilient thin web of metal. In one embodiment, the first and second hook features can be shaped and sized to closely conform to an outer diameter and extending ridge and/or external ears of the catheter hub. In one embodiment, the safety clip can further include a proximal wall to which the first and second guard arms are operably coupled. In one embodiment, the proximal wall can include an aperture sized to enable the elongate body of the needle cannula to pass therethrough, and inhibit passage of a needle feature of the needle cannula therethrough. In one embodiment, and the second position, proximal movement of the sharp distal tip relative to the safety clip can be inhibited by interference between the needle feature and the aperture of the proximal wall, and distal movement of the sharp distal tip relative to the safety clip can be inhibited by the unaligned first and second apertures of the retention tabs. In one embodiment, one or more safety clip retention tabs can be operably coupled to the proximal wall. In one embodiment the one or more safety clip retention tabs can be constructed of a resilient material and can extend from the proximal wall at an oblique angle. In one embodiment the one or more safety clip retention tabs can be configured to flex to enable the safety clip to be inserted into a chamber of the housing, and inhibit removal of the safety clip from the chamber once inserted.

Another embodiment of the present disclosure provides a catheter insertion device configured to inhibit release of a catheter hub from the catheter insertion device until a sharp distal tip of a needle cannula utilized to insert a catheter assembly is safely captured in a needle clip, thereby reducing the risk of an inadvertent needle stick. The catheter insertion device can include a needle cannula, handle, and safety clip. The needle cannula can have a sharp distal tip and a proximal end. The handle can be operably coupled to the proximal end of the needle cannula. The safety clip assembly can include a housing and a safety clip. The safety clip can include first and second guard arms configured to selectively grip a catheter hub of a catheter assembly. Each guard arm can include a respective first and second retention tab including structure defining a respective first and second aperture configured to enable passage of the needle catheter therethrough. The safety clip assembly can be configured to transition between a first position, in which the first and second guard arms are restrained against their natural bias by the passage of a needle cannula through an alignment of the first and second apertures of the first and second retention tabs, such that the first and second hook features are in gripping contact with the catheter hub, and a second position, in which the needle cannula is proximally withdrawn through the first and second apertures to enable the first and second guard arms to be naturally biased apart to release gripping contact with the catheter hub, such that the first and second apertures of the first and second retention tabs become unaligned to trap the sharp distal tip of the needle cannula within the safety clip.

The summary above is not intended to describe each illustrated embodiment or every implementation of the present disclosure. The figures and the detailed description that follow more particularly exemplify these embodiments.

### BRIEF DESCRIPTION OF THE DRAWINGS

The disclosure can be more completely understood in consideration of the following detailed description of various embodiments of the disclosure, in connection with the accompanying drawings, in which:
FIG. 1A is a perspective view depicting a conventional catheter insertion assembly, in which a catheter is coaxially positioned over a catheter insertion device.
FIG. 1B is a perspective view depicting the conventional catheter insertion assembly of FIG. 1A, in which the catheter is removed from the catheter insertion device.
FIG. 2A is a perspective view depicting a safety catheter insertion assembly in accordance with an embodiment of the disclosure.
FIG. 2B is an exploded, perspective view depicting the safety catheter assembly of FIG. 2A.
FIG. 3 is a close-up, partial, perspective view depicting a needle transition positioned on a needle cannula, in accordance with an embodiment of the disclosure.
FIG. 4A is a perspective view depicting a safety catheter assembly including a closed system catheter assembly in accordance with an embodiment of the disclosure.
FIG. 4B is an exploded, perspective view depicting the safety catheter assembly of FIG. 4A.
FIG. 5 is a partial, cross-sectional view depicting a safety catheter insertion assembly including a blood control feature, in accordance with an embodiment of the disclosure.
FIG. 6 is a partial, cross-sectional view of a safety catheter insertion assembly including a side port, in accordance with an embodiment of the disclosure.
FIG. 7A is a perspective view depicting a safety catheter assembly in a first or ready for use position, in accordance with an embodiment of the disclosure.
FIG. 7B is a perspective view depicting the safety catheter assembly of FIG. 7A in a second or safe position, in accordance with an embodiment of the disclosure.
FIG. 8A is a partial, cross-sectional, profile view depicting a safety catheter assembly in a first or ready for use position, in accordance with an embodiment of the disclosure.
FIG. 8B is a partial, cross-sectional, profile view depicting the safety catheter assembly of FIG. 8A, in which the safety catheter assembly is rotated 90° about a longitudinal axis of the needle cannula.
FIG. 9A is a partial, perspective view depicting a needle cannula, safety clip, and catheter hub of a safety catheter insertion device in a first or ready for use position, in accordance with an embodiment of the disclosure.
FIG. 9B is a partial, perspective view depicting the needle cannula, safety clip, and catheter hub of FIG. 9A in a second or safe position, in accordance with an embodiment of the disclosure.
FIG. 10A is a profile view depicting a safety clip in a first or ready for use position, in accordance with an embodiment of the disclosure.
FIG. 10B is a profile view depicting the safety clip of FIG. 10A in a second or safe position, in accordance with an embodiment of the disclosure.

While embodiments of the disclosure are amenable to various modifications and alternative forms, specifics thereof shown by way of example in the drawings will be described in detail. It should be understood, however, that the intention is not to limit the disclosure to the particular embodiments described.

### DETAILED DESCRIPTION

Various example embodiments of catheters are described herein for use in accessing the vein of a subject. It is to be appreciated, however, that the example embodiments described herein can alternatively be used to access the vascular of a subject at locations other than a vein, including but not limited to the artery of a subject. It is additionally to be appreciated that the term "clinician" refers to any individual that can perform a catheter insertion procedure with any of the example embodiments described herein or alternative combinations thereof. Similarly, the term "subject," as used herein, is to be understood to refer to an individual or object in which the catheter is to be inserted, whether human, animal, or inanimate. Various descriptions are made herein, for the sake of convenience, with respect to the procedures being performed by a clinician to access the vein of a subject, while the disclosure is not limited in this respect.

It is also to be appreciated that the term "distal," as used herein, refers to the direction along an axis that lies parallel to a needle cannula of a safety catheter assembly that is closest to the subject during catheter insertion. Conversely, the term "proximal," as used herein, refers to the direction lying along the axis parallel to the needle cannula that is further away from the subject when the catheter is inserted into the vein of the subject, opposite to the distal direction.

Referring to FIGS. 1A-B, a conventional catheter insertion assembly 50 is depicted. Details of the conventional catheter insertion assembly 50 are described in the Background section above.

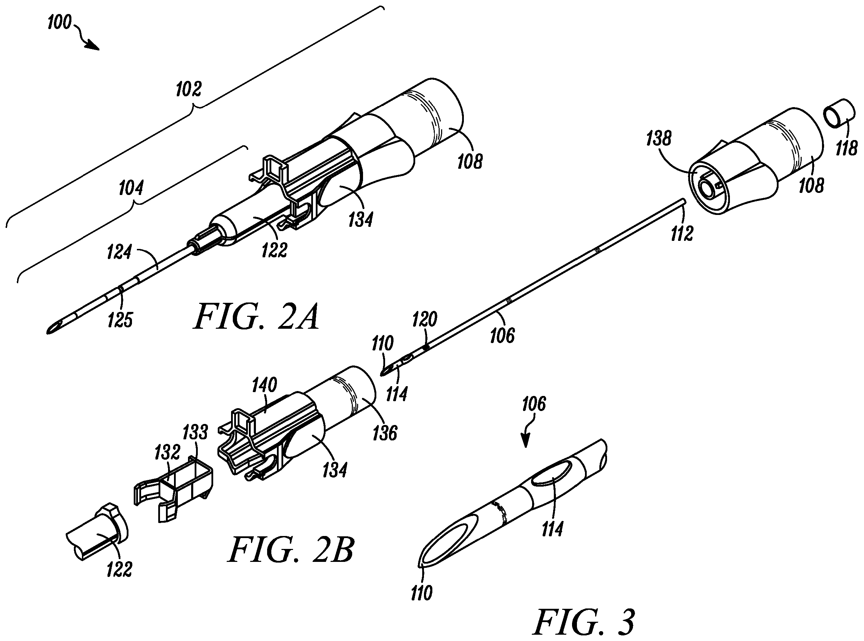

Referring to FIG. 2A, a perspective view of a safety catheter insertion assembly 100 is depicted in accordance with an embodiment of the disclosure. FIG. 2B depicts an exploded view of the safety catheter insertion assembly 100 of FIG. 2A. The safety catheter assembly 100 can include a catheter insertion device 102 and a catheter assembly 104. The catheter insertion device 102 can include an insertion or needle cannula 106 operably coupled to a handle 108. The needle cannula 106 can include an elongate cylindrically shaped metal structure defining a lumen that extends between a sharpened distal needle tip 110 and a proximal end 112. The sharp distal needle tip 110 can be constructed and arranged to pierce the skin of a subject during catheter insertion. For example, in one embodiment, the sharp distal tip 110 can include a V-point designed to reduce the penetration force used to penetrate the needle 106 and a portion of the catheter insertion assembly 104 through the skin, tissue, and vein wall of a subject. In one embodiment, the length of the needle 106 can be extended to aid in the insertion of the catheter assembly 104 into obese patients.

The needle cannula 106 can further include a transition 114 having a different cross-sectional size and are shaped then other portions of the needle 106 positioned proximal to the transition 114. Referring to FIG. 3, a close-up, partial, perspective view of a transition 114 positioned on a needle cannula 106 is depicted in accordance with an embodiment of the disclosure. Needle transition 114 (alternatively referred to as a needle bump or cannula bump) can be created by crimping opposed sides of the needle cannula 106, or otherwise disrupting the structure of the needle 106, so that the outer surfaces of the needle 106 extend to a larger radial position than other portions of the needle cannula 106, as measured from the center of the needle axis. Needle transitions 114 can be formed differently, according to alternative embodiments, such as by adding material to the exterior of the needle, among other ways.

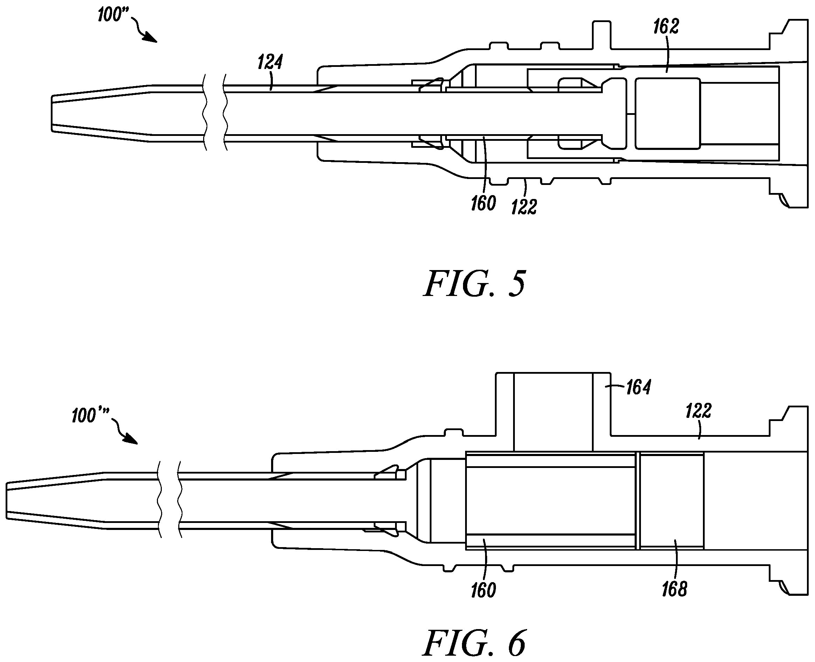

The proximal end 112 of the needle cannula 106 can be operably coupled to the handle 108. The handle 108 can include a gripping portion for manipulation by a clinician. In one embodiment, the catheter insertion device 102 can be constructed to provide a visual indication of flashback when the sharpened distal tip 110 of the needle 106 enters the vein of a subject. In this embodiment, the handle 108 can include a flash chamber 116 (as depicted in FIG. 6A-B) in fluid communication with the lumen of the needle. For example, in some embodiments, the flash chamber 116 can be integrally molded together with the handle 108, such that the flash chamber 116 is at least partially surrounded by the handle 108. In other embodiments, the flash chamber 116 can be a separate component housed within at least a portion of the handle 108.

When the sharp distal tip 110 enters a vein during catheter insertion, blood or bodily fluid enters the needle lumen from the vein and flows proximally through the needle 106 into the flash chamber 116. The flash chamber 116 can be sealed at one end by a flash plug 118. The flash plug 118 can be made out of an air permeable, hydrophilic material that enables the passage of air, but inhibits the passage of liquid. For example, in one embodiment, the flash plug 118 can include a plurality of pores shaped and sized to enable the passage of low-pressure gas, but Inhibits the passage of low-pressure liquid, such that pores of the flash plug 118 become effectively sealed upon contact with the low-pressure liquid. Air that resides in the needle lumen and flash chamber 116 is therefore pushed through the flash plug 118 by the incoming blood, until the blood reaches the flash plug 118 or is otherwise stopped. In some embodiments, the handle 108, or portions thereof, can be constructed of a clear or translucent material configured to enable a clinician to view the presence of blood within the flash chamber 116. In this respect, the clinician can be alerted when the needle has entered the vein of the subject by the presence of blood within the flash chamber 116.

In one embodiment, features of the catheter insertion device 102, other than a flash chamber 116, can provide an indication that the sharp distal tip 110 has entered the vein of a subject. For example, the needle cannula 106 can include a notch 120. In this embodiment, blood flow enters the needle lumen when the sharpened distal tip 110 enters the vein. As blood flows proximately in the needle lumen, some blood passes through the notch 120 and into an annular space that lies between an exterior of the needle 106 and an interior of the catheter assembly 104. The presence of blood in the annular space can be viewed by a clinician through a clear or translucent portion of the catheter assembly 104, thereby providing an indication that the sharpened distal tip 110 is present in a vein.

As further depicted, the catheter insertion device 102 can additionally include a safety clip 132 and a safety clip housing 134. In some embodiments, the safety clip housing 134 can include a proximal portion 136 shaped and sized to mate with a distal portion 138 of the handle 108. For example, in one embodiment, the proximal portion 136 can include an extension configured to matingly fit within a corresponding socket defined within the distal portion 138 of the handle 108.

The safety clip 132 can be selectively retained within a distal portion 140 of the safety clip housing 134. For example, in one embodiment, the distal portion 140 of the safety clip housing 134 can define a safety clip chamber 142 configured to at least partially house the safety clip 132. In some embodiments, the safety clip 132 can include one or more retention tabs 133 configured to create an interference fit with a portion of the safety clip chamber 142, so as to aid in the retention of the safety clip 132 within the safety clip housing 134.

The catheter assembly 104 can include a catheter hub 122 and a catheter tube 124. In one embodiment, the catheter tube 124 can extend from a tapered distal end to a proximal end, where the catheter tube 124 can be operably coupled to the catheter hub 122. The catheter tube 124 can define a lumen configured to provide a fluid pathway between a vein of a subject and the catheter hub 122. In one embodiment, the catheter tube 124 can include a barium radio opaque line to ease in the identification of the catheter tube 124 during radiology procedures. In some embodiments, the catheter tube 124 can include a necked-down portion 125 configured to provide a two-stage visual indication during catheter insertion into a vein of a patient. One example a necked-down portion on a catheter tube 124 is disclosed in U.S. Provisional App. No. 62/575,045 (filed October 20, 2017).

The catheter hub 122 can include a catheter hub body having a distal end, a proximal end and an internal wall defining an interior cavity therebetween. The interior cavity can include a proximal portion extending from the open proximal end, and a distal portion in closer proximity to the distal end. In one embodiment, the distal end of the catheter hub body is operably coupled to the proximal end of the catheter tube 124, such that the lumen of the catheter tube 124 is in fluid communication with the proximal portion of the interior cavity. In some embodiments, the proximal portion of the interior cavity can be shaped according to luer taper standards, so as to matingly receive a luer taper. The proximal end of the catheter hub body can also be provided with external ears, or the like, to secure the luer taper in the catheter hub 122, such as when the luer taper is coupled with a male luer lock collar of an administration set or syringe.

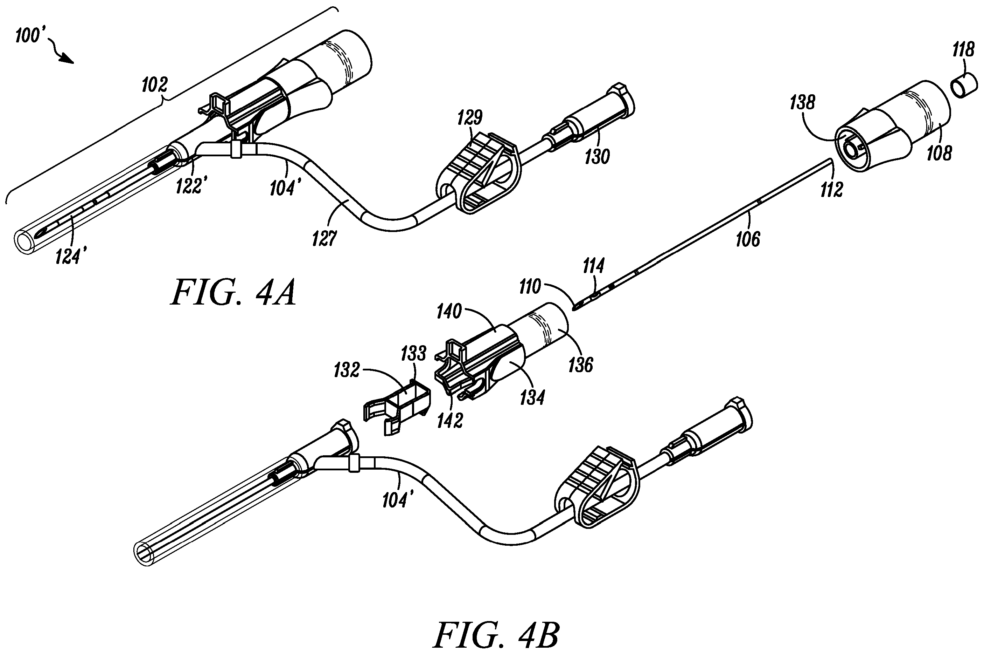

In some embodiments, the catheter assembly can comprise a closed system catheter assembly 104'. Referring to FIG. 4A, a perspective view of a safety catheter insertion assembly 100' including a closed system catheter assembly 104' is depicted in accordance with an embodiment of the disclosure. FIG. 2B depicts an exploded view of the safety catheter insertion assembly 100' of FIG. 4A. In one embodiment, the closed system catheter assembly 104' can include a catheter hub 122', catheter tube 124', extension tube 126, an extension tube clamp 128, and a needleless connector 130. Some embodiments can further include a wing assembly and/or vent cap (not depicted). One such example of a closed system catheter assembly 104' is disclosed in U.S. Patent Publ. No. 2017/0239443.

In some embodiments, the catheter assembly 104 can further include a blood control feature configured to inhibit blood from escaping after withdrawal of the needle cannula 106, thereby reducing the risk of exposure of blood or other bodily fluids to clinicians, particularly a consideration of sensitivity where blood-borne diseases may be present. Referring to FIG. 5, a partial cross sectional view of a safety catheter insertion assembly 100" including a blood control feature is depicted in accordance with an embodiment of the disclosure. In one embodiment, the safety catheter insertion assembly 100" can include an actuator 160 secured to the distal end of the catheter hub 122, so as to extend axially within the interior cavity. In one embodiment, the proximal end of the catheter tube 124 can be secured within the interior cavity of the catheter hub 122 with the aid of the actuator 160.

A seal member 162, alternatively referred to as a blood control valve, can also be secured within the interior cavity of the catheter hub 122 with the aid of the actuator 160, such that the seal member 162 is axially shiftable relative to the actuator 160 between a closed or sealed position in which flow of bodily fluid from the catheter tube 124 into the interior cavity of the catheter hub 122 is inhibited or restricted, and an open or actuated position, in which the seal member 162 is shifted relative to the actuator 160 thereby enabling the flow of bodily fluid from the catheter tube 124 into a proximal portion of the interior cavity of the catheter hub 122. Thus, the actuator 160 functions to both secure the catheter tube 124 to the catheter hub 122, and to support the seal member 162. One example of such a blood control feature is disclosed in U.S. Patent No. 9,545,495.

In some embodiments, the catheter assembly 104 can include a side port. Referring to FIG. 6, a partial cross sectional view of a safety catheter insertion assembly 100‴ including a side port 164 is depicted in accordance with an embodiment of the disclosure. In one embodiment, the interior wall of the catheter hub 122 can define a side port aperture 164 configured to enable an alternative fluid communication path with the interior cavity of the catheter hub 122. In one embodiment, the side port 164 can be positioned substantially orthogonal to a longitudinal axis of the catheter hub 122.

The side port 164 can be selectively sealed by a flexible sealing membrane 166 positioned within the interior cavity of the catheter hub 122. The sealing membrane 166 can be configured to deform when a sufficient fluid pressure is applied to the side port 164 from outside of the catheter hub 122, thereby enabling a flow of fluid into the interior cavity of the catheter hub 122. A septum or valve 168 can be positioned proximal to the sealing member 166 to inhibit fluid entering the interior cavity of the catheter hub 122 from the side port 164 from escaping out of the proximal end of the catheter hub 122. An example of such a septum or valve 168 is disclosed in U.S. Patent Publ. No. 2017/0239443.

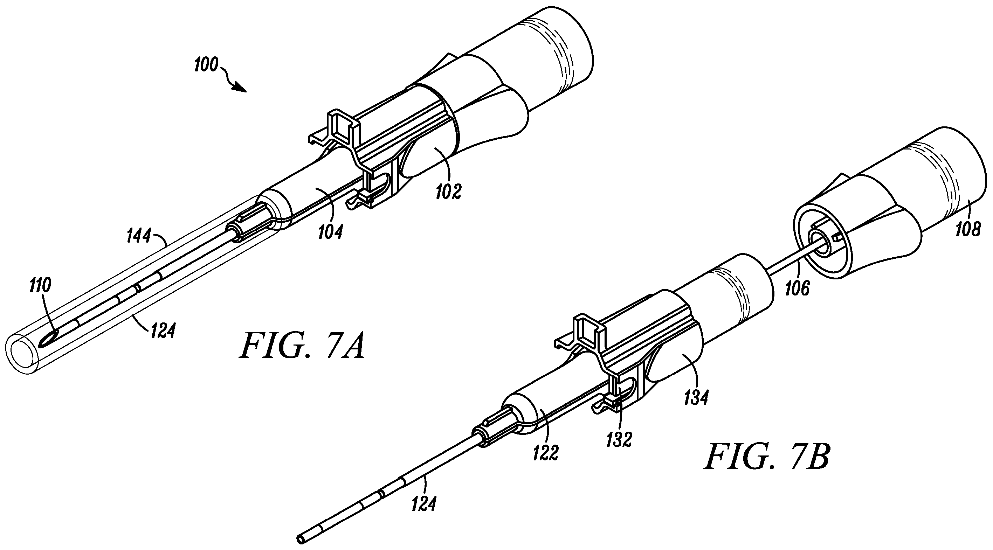

As depicted in FIG. 7A, the safety catheter insertion assembly 100 can be provided in the first or ready for use position, in which the catheter assembly 104 is connected to the catheter insertion device 102. In particular, the catheter tube 124, can be positioned over the needle cannula 106 of the catheter insertion device 102, with a sharp distal tip 110 of the needle 106 protruding from the distal end of the catheter tube 124. In some embodiments, the safety catheter assembly 100 can be provided for use in a sterilized and assembled state, contained within a sealed package.

To insert the catheter into the vein of a subject, a clinician first removes the safety catheter assembly 100 from the packaging. A needle sheath 144 covering the needle cannula 106 can be removed to expose the sharp distal tip 110 of the needle cannula 106. The clinician then punctures an identified site on the patient or subject with the sharp distal tip 110 and urges the needle cannula 106 forward until the sharp distal tip 110 and a portion of the catheter tube 124 enters the vein of the subject.

The catheter assembly 104 can then be moved distally over the needle 106, threading the catheter assembly 104 into the vein of the subject as the catheter insertion device 102 is held stationary. With the catheter assembly 104 positioned as desired, the clinician can withdraw the needle cannula 106 from the patient's vein by pulling the catheter insertion device 102 proximally away from the subject while holding the catheter assembly 104 generally stationary with respect to the subject. The catheter insertion device 102 is pulled proximally until the needle cannula 106 is separated from the catheter assembly 104.

As depicted in FIG. 7B, the catheter insertion device 102 can be released from the catheter assembly 104 according to a second or safe position. In some embodiments, as the catheter insertion device 102 transitions from the first or ready for use position to the second or safe position, the safety clip 132 and safety clip housing 134 are shifted distally away from the handle 108 along a longitudinal axis of the needle cannula 106. Further, in some embodiments, the safety clip 132 can be positioned over the sharp distal tip 110 prior to a release of the catheter insertion device 102 from the catheter assembly 104 for the purpose of inhibiting unwanted needle sticks. In the safe position, the clinician can dispose of the catheter insertion device 102 in a sharps container.

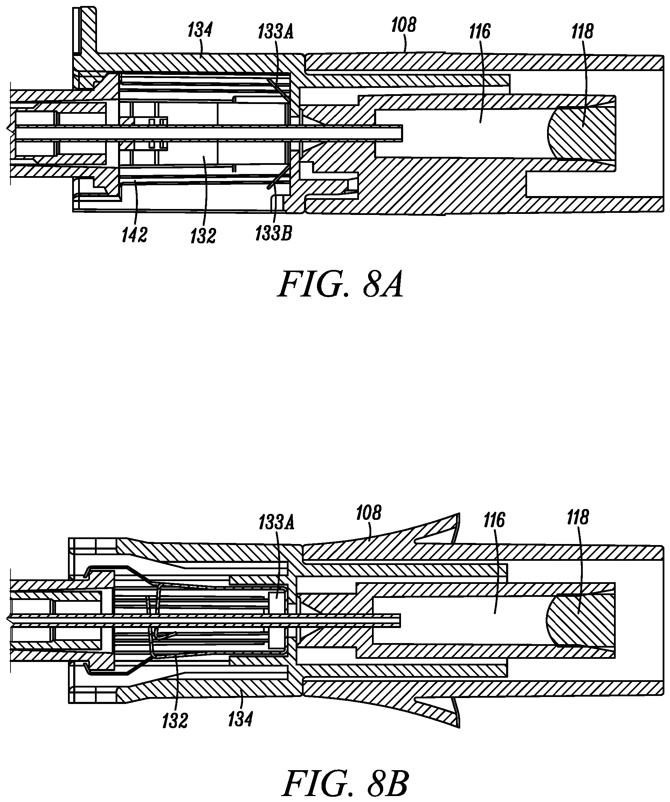

Referring to FIG. 8A, a partial, cross-sectional, profile view of a safety catheter assembly 100 in a first or ready for use position, is depicted in accordance with an embodiment of the disclosure. FIG. 8B depicts, a partial, cross-sectional, profile view of the safety catheter assembly 100 of FIG. 8A, in which the safety catheter assembly 100 is rotated 90° about the longitudinal axis of the needle cannula 106. In one embodiment, the safety clip 132 can be selectively retained within a portion of the safety clip housing 134 via the aid of one or more retention tabs 133A/B. For example, in one embodiment, the one or more retention tabs 133A/B of the safety clip 132 can be configured to deflect when inserting the safety clip 132 within the chamber 142 of the safety clip housing 134, thereby enabling the safety clip 132 slide into position within the safety clip housing 134. Distal movement of the safety clip 132 out of the chamber 142 of the safety clip housing 134 can be inhibited by an interference fit between the one or more retention tabs 133A/B and one or more walls defining the chamber 142. In this manner, the retention tabs 133A/B effectively lock the safety clip 132 within the safety clip housing 134 after assembly.

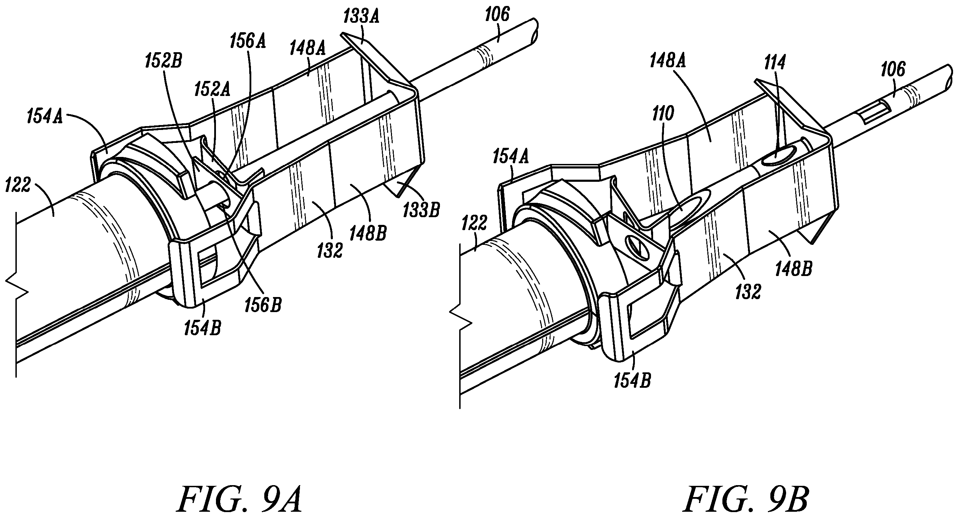

The safety clip 134 can be configured to actually slide along the needle cannula 106 between a first or ready for use position (as depicted in FIG. 9A), in which the needle cannula 106 traverses through the safety clip 134 and a portion of the safety clip 134 grips the catheter hub 122, and a second or safe position (as depicted in FIG. 9B), in which the sharp distal tip 110 is captured within the safety clip 132 for the purpose of inhibiting unwanted needle sticks.

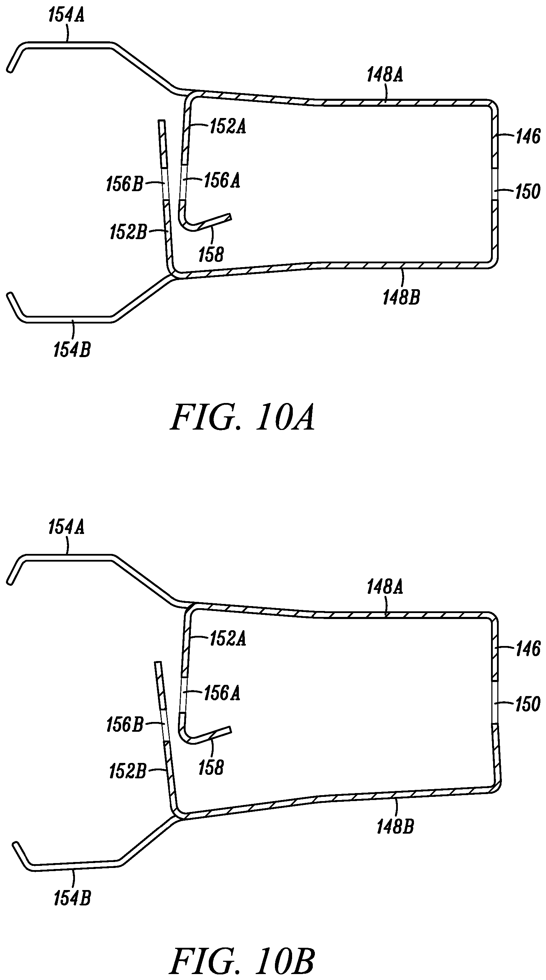

Referring to FIGS. 10A-B, an embodiment of the safety clip 132 is depicted in accordance with an embodiment of the disclosure. FIG. 10A depicts the safety clip 132 in the first or ready for use position, in which a needle cannula 106 can pass through a portion of the safety clip 132, so as to position the safety clip 132 in a gripping position around a catheter hub 122. FIG. 10B depicts the safety clip 132 in an expanded, second or safe position, in which a sharp distal tip 110 of the needle cannula 106 can be captured within the safety clip 132, and the safety clip 132 can resume its natural, unbiased state to release its gripping position around the catheter hub 122.

In one embodiment, the safety clip 128 can include a proximal wall 146 and one or more guard arms 148. The proximal wall 146 can define an aperture 150 configured to be positioned around or over the needle cannula 106. In some embodiments, the proximal wall 146 and the aperture 150 can be configured to engage the needle transition 114 to inhibit distal advancements of the safety clip 134 off of the sharp distal tip 110 of the needle cannula 106. In one embodiment, the surface of the proximal wall 146 can be substantially orthogonal to the longitudinal axis of the needle cannula 106. In other embodiments, the surface can be positioned at an oblique or acute angle with respect to the longitudinal axis of the needle cannula 106.

The one or more guard arms 148 can extend distally from the proximal wall 146. In one embodiment, the safety clip 132 can include a pair of guard arms 148A/B. In one embodiment, the guard arms 148A/B can further define a retention tab 152A/B and a hook feature 154A/B. Each retention tab 152 can further define an aperture 156A/B configured to be positioned around or over the needle cannula 106.

The safety 132 128 can be formed of a generally resilient material, for example medical grade stainless steel, such that guard arms 148A/B have a natural bias towards a free or relaxed state (as depicted in FIG. 10B). For example, in one embodiment, the safety clip 132 is in a free state when the safety clip 132 is in the second or safe position. Accordingly, in this embodiment, when the safety clip 132 is in the first or ready for use position, and the needle cannula 106 passes through the aligned apertures 156A/B of the retention tabs 152A/B, the guard arms 148A/B are biased against the needle cannula 106, as the presence of the needle cannula 106 causes the guard arms 148A-B to be deflected away from their free state. When the needle cannula 106 is retracted, and the sharp distal tip 110 moves proximally between the guard arms 148 out of the apertures 156A/B, the guard arms 148 naturally bias towards their free state, such that the apertures 156A/B become unaligned, thereby inhibiting the sharp distal tip 110 from reemergence from the apertures 156A/B.

In some embodiments, further proximal movement of the sharp distal tip 110 is inhibited by a cross dimension of the needle transition 114 being larger than the aperture 150 in the proximal wall 146 of the safety clip 132. Distal movement of the sharp distal tip 110 is inhibited by the retention tabs 152A/B. In particular, in one embodiment, distal movement of the sharp distal tip 110 is inhibited by the apertures 156A/B becoming unaligned. In one embodiment, at least one of the retention tabs 152A can further include a hook portion 158 configured to inhibit the needle cannula 106 from traversing around an outer edge of the retention tab 152A to pass through aperture 156B.

In one embodiment, the safety clip 134 operates as a "passive release mechanism" The term passive release mechanism, as used herein, as understood to refer to features of a catheter insertion assembly 100 that inhibit the release of the catheter assembly 104 from the needle insertion device 102 until after the sharp distal tip 110 of the needle cannula 106 has been captured within the safety clip 134. Some or all of the features of the passive release mechanism can be integral with other components of the catheter insertion assembly 100. In this respect, the term passive release mechanism does not necessarily refer to a component that is separate from the catheter insertion device 102 and/or the catheter assembly 104. Rather, it is to be appreciated that various components of the catheter insertion device 102 and/or catheter assembly 104 can form the passive release mechanism.

In one embodiment, the passive release mechanism can be configured to couple the catheter hub 122 to the catheter insertion device 102 in the first or ready for use position, and release the catheter hub 122 from the catheter insertion device 102 in the second or safe position. Further, the passive release mechanism inhibits release of the catheter hub 122 from the catheter insertion 102 device until after the sharp distal tip 110 of the needle cannula 106 is in a safe position, where access to the sharp distal tip 110 is inhibited. Release of the catheter hub 122 from the catheter insertion device 102 can occur during a catheter insertion procedure without a need to perform additional steps aside from safely retracting the needle cannula 106. In this respect, the catheter can be "passively" released by a clinician to obtain passive safety. By way of example, the catheter can be released when a clinician pulls on a portion of the catheter insertion device 102 as the clinician withdraws the needle 106 from the catheter assembly 104.

It should be understood that the individual steps used in the methods of the present teachings may be performed in any order and/or simultaneously, as long as the teaching remains operable. Furthermore, it should be understood that the apparatus and methods of the present teachings can include any number, or all, of the described embodiments, as long as the teaching remains operable.

Persons of ordinary skill in the relevant arts will recognize that embodiments may comprise fewer features than illustrated in any individual embodiment described above. The embodiments described herein are not meant to be an exhaustive presentation of the ways in which the various features may be combined. Accordingly, the embodiments are not mutually exclusive combinations of features; rather, embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted. Although a dependent claim may refer in the claims to a specific combination with one or more other claims, other embodiments can also include a combination of the dependent claim with the subject matter of each other dependent claim or a combination of one or more features with other dependent or independent claims. Such combinations are proposed herein unless it is stated that a specific combination is not intended. Furthermore, it is intended also to include features of a claim in any other independent claim even if this claim is not directly made dependent to the independent claim.

Moreover, reference in the specification to "one embodiment," "an embodiment," or "some embodiments" means that a particular feature, structure, or characteristic, described in connection with the embodiment, is included in at least one embodiment of the teaching. The appearances of the phrase "in one embodiment" in various places in the specification are not necessarily all referring to the same embodiment.

## Claims

1. A catheter insertion device (102) configured to inhibit release of a catheter hub (122) of a catheter assembly (104) from the catheter insertion device until a sharp distal tip (110) of a needle cannula (106) utilized to insert the catheter assembly (104) is safely captured in a needle clip (132), the catheter insertion device (102) comprising:
a needle cannula (106) having a sharp distal tip (110) and a proximal end (112);
a handle (108) operably coupled to the proximal end (112) of the needle cannula (106); and
a safety clip assembly including a housing (134) and a safety clip (132), the safety clip (132) including first and second guard arms (148A, 148B) extending from a proximal wall (146), the first and second guard arms (148A, 148B) having respective first and second hook features (154A, 154B) configured to selectively grip the catheter hub (122) of the catheter assembly (104) and respective needle tip retention tabs (152A, 152B) having corresponding first and second apertures (156A, 156B) configured to enable passage of the needle cannula (106) therethrough,
wherein the safety clip assembly is configured to be in a first position in which the first and second guard arms (148A, 148B) are restrained against their natural bias by the passage of the needle cannula (106) through the first and second apertures (156A, 156B) of the first and second retention tabs (152A, 152B) such that the first and second hook features (154A, 154B) extending from respective distal ends of guard arms (148A, 148B) are in gripping contact with the catheter hub (122); and
wherein the safety clip assembly is configured to be in a second position in which the needle cannula (106) is proximally withdrawn through the first and second apertures (156A, 156B) to enable the first and second guard arms (148A, 148B) to be naturally biased apart to release the catheter hub (122) from the gripping contact by the first and second hook features (154A, 154B) and to return the retention tabs (152A, 152B) to their natural position to inhibit distal movement of the sharp distal tip (110) of the needle cannula (106) within the safety clip (132), **characterised by** the safety clip (132) including two opposed safety clip retention tabs (133A, 133B) extending from sides of the proximal wall (146) different from where the first and second guard arms (148A, 148B) extend from the proximal wall (146), the two opposed safety clip retention tabs (133A, 133B) configured to deflect when the safety clip (132) is inserted into a chamber (142) of the housing (134) to enable the safety clip (132) to be positioned at least partially within the housing (134) and to provide an interference fit between one or more of the two opposed safety clip retention tabs (133A, 133B) and one or more of the walls defining the chamber (142) of the housing (134) to inhibit distal movement of the safety clip (132) out of the housing (134).

2. The catheter insertion device of claim 1, wherein the safety clip (132) is constructed of a resilient thin web of metal.

3. The catheter insertion device of claim 1, wherein the first and second hook features (154A, 154B) of the first and second guard arms (148A, 148B), respectively, are shaped and sized to closely conform to an outer diameter and extending ridge and/or external ears of the catheter hub (122).

4. The catheter insertion device of claim 1, wherein the first and second guard arms (148A, 148B) are operably coupled to the proximal wall (146).

5. The catheter insertion device of claim 4, wherein the proximal wall (146) includes an aperture (150) configured to enable the elongate body of the needle cannula (106) to pass therethrough, but inhibits passage of a needle feature (114) of the needle cannula (106) therethrough.

6. The catheter insertion device of claim 5, wherein in the second position, the sharp distal tip (110) of the needle cannula (106) is captured in the safety clip (132) as proximal movement of the sharp distal tip (110) relative to the safety clip (132) is inhibited by interference between the needle feature (114) and the aperture (150) of the proximal wall (146) and distal movement of the sharp distal tip (110) relative to the safety clip (132) is inhibited by the retention tabs (152A, 152B).

7. The catheter insertion device of claim 1, wherein the safety clip retention tabs (153A, 153B) are constructed of a resilient material and extend from the proximal wall (146) at an oblique angle.

## Patentansprüche

1. Kathetereinführungsvorrichtung (102), die dazu ausgestaltet ist, das Lösen eines Katheteranschlusses (122) einer Katheteranordnung (104) von der Kathetereinführungsvorrichtung zu verhindern, bis eine scharfe distale Spitze (110) einer Nadelkanüle (106), die zum Einführen der Katheteranordnung (104) genutzt wird, sicher in einem Nadelclip (132) festgehalten wird, wobei die Kathetereinführungsvorrichtung (102) umfasst:
eine Nadelkanüle (106), die eine scharfe distale Spitze (110) und ein proximales Ende (112) aufweist;
einen Griff (108), der betriebsfähig an das proximale Ende (112) der Nadelkanüle (106) gekoppelt ist; und
eine Sicherheitsclipanordnung, die ein Gehäuse (134) und einen Sicherheitsclip (132) umfasst, wobei der Sicherheitsclip (132) einen ersten und einen zweiten Schutzarm (148A, 148B) umfasst, die sich von einer proximalen Wand (146) erstrecken, wobei der erste und der zweite Schutzarm (148A, 148B) ein jeweiliges erstes und zweites Hakenmerkmal (154A, 154B), die dazu ausgestaltet sind, den Katheteranschluss (122) der Katheteranordnung (104) selektiv zu greifen, und jeweilige Nadelspitzen-Rückhaltelaschen (152A, 152B) aufweisen, die entsprechende erste und zweite Öffnungen (156A, 156B) aufweisen, die dazu ausgestaltet sind, den Durchgang der Nadelkanüle (106) dort hindurch zu ermöglichen,
wobei die Sicherheitsclipanordnung dazu ausgestaltet ist, sich in einer ersten Position zu befinden, in welcher der erste und der zweite Schutzarm (148A, 148B) durch den Durchgang der Nadelkanüle (106) derart durch die erste und die zweite Öffnung (156A, 156B) der ersten und der zweiten Rückhaltelasche (152A, 152B) gegen ihre natürliche Vorspannung zurückgehalten werden, dass das erste und das zweite Hakenmerkmal (154A, 154B), die sich von jeweiligen distalen Enden von Schutzarmen (148A, 148B) erstrecken, in Greifkontakt mit dem Katheteranschluss (122) stehen; und
wobei die Sicherheitsclipanordnung dazu ausgestaltet ist, sich in einer zweiten Position zu befinden, in der die Nadelkanüle (106) proximal durch die erste und die zweite Öffnung (156A, 156B) zurückgezogen wird, um es dem ersten und dem zweiten Schutzarm (148A, 148B) zu ermöglichen, natürlich auseinander vorgespannt zu werden, um den Katheteranschluss (122) aus dem Greifkontakt durch das erste und das zweite Hakenmerkmal (154A, 154B) zu lösen und die Rückhaltelaschen (152A, 152B) in ihre natürliche Position zurückzubringen, um distale Bewegung der scharfen distalen Spitze (110) der Nadelkanüle (106) innerhalb des Sicherheitsclips (132) zu verhindern, **dadurch gekennzeichnet, dass** der Sicherheitsclip (132) zwei gegenüberliegende Sicherheitsclip-Rückhaltelaschen (133A, 133B) umfasst, die sich von Seiten der proximalen Wand (146) erstrecken, die sich von dort unterscheiden, wo der erste und der zweite Schutzarm (148A, 148B) sich von der proximalen Wand (146) erstrecken, wobei die zwei gegenüberliegenden Sicherheitsclip-Rückhaltelaschen (133A, 133B) dazu ausgestaltet sind, auszulenken, wenn der Sicherheitsclip (132) in eine Kammer (142) des Gehäuses (134) eingeführt wird, um das Positionieren des Sicherheitsclips (132) zumindest teilweise innerhalb des Gehäuses (134) zu ermöglichen und eine Presspassung zwischen einer oder mehreren der zwei gegenüberliegenden Sicherheitsclip-Rückhaltelaschen (133A, 133B) und einer oder mehreren von den Wänden bereitzustellen, welche die Kammer (142) des Gehäuses (134) definieren, um distale Bewegung des Sicherheitsclips (132) aus dem Gehäuse (134) zu verhindern.

2. Kathetereinführungsvorrichtung nach Anspruch 1, wobei der Sicherheitsclip (132) aus einem nachgiebigen dünnen Gewebe aus Metall aufgebaut ist.

3. Kathetereinführungsvorrichtung nach Anspruch 1, wobei das erste und das zweite Hakenmerkmal (154A, 154B) des ersten beziehungsweise zweiten Schutzarms (148A, 148B) derart geformt und bemessen sind, dass sie sich eng an einen äußeren Durchmesser und eine sich erstreckende Rippe und/oder äußere Ösen des Katheteranschlusses (122) anpassen.

4. Kathetereinführungsvorrichtung nach Anspruch 1, wobei der erste und der zweite Schutzarm (148A, 148B) betriebsfähig an die proximale Wand (146) gekoppelt sind.

5. Kathetereinführungsvorrichtung nach Anspruch 4, wobei die proximale Wand (146) eine Öffnung (150) umfasst, die dazu ausgestaltet ist, es dem länglichen Körper der Nadelkanüle (106) zu ermöglichen, sie zu durchqueren, aber ihr Durchqueren durch ein Nadelmerkmal (114) der Nadelkanüle (106) verhindert.

6. Kathetereinführungsvorrichtung nach Anspruch 5, wobei die scharfe distale Spitze (110) der Nadelkanüle (106) in der zweiten Position in dem Sicherheitsclip (132) festgehalten wird, während proximale Bewegung der scharfen distalen Spitze (110) in Bezug auf den Sicherheitsclip (132) durch gegenseitige Beeinflussung zwischen dem Nadelmerkmal (114) und der Öffnung (150) der proximalen Wand (146) verhindert wird und distale Bewegung der scharfen distalen Spitze (110) in Bezug auf den Sicherheitsclip (132) durch die Rückhaltelaschen (152A, 152B) verhindert wird.

7. Kathetereinführungsvorrichtung nach Anspruch 1, wobei die Sicherheitsclip-Rückhaltelaschen (153A, 153B) aus einem nachgiebigen Material aufgebaut sind und sich in einem schrägen Winkel von der proximalen Wand (146) erstrecken.

## Revendications

1. Dispositif d'insertion de cathéter (102) configuré pour empêcher la libération d'un raccord de cathéter (122) d'un ensemble de cathéter (104) du dispositif d'insertion de cathéter jusqu'à ce qu'une pointe distale pointue (110) d'une canule d'aiguille (106) utilisée pour insérer l'ensemble de cathéter (104) soit capturée, en toute sécurité, dans une attache d'aiguille (132), le dispositif d'insertion de cathéter (102) comprenant :
une canule d'aiguille (106) ayant une pointe distale pointue (110) et une extrémité proximale (112) ;
une poignée (108) couplée, de manière opérationnelle, à l'extrémité proximale (112) de la canule d'aiguille (106) ; et
un ensemble d'attache de sécurité comprend un boîtier (134) et une attache de sécurité (132), l'attache de sécurité (132) comprenant des premier et second bras de protection (148A, 148B) s'étendant à partir d'une paroi proximale (146), les premier et second bras de protection (148A, 148B) ayant des première et seconde caractéristiques de crochet (154A, 154B) configurées pour saisir, sélectivement, le raccord de cathéter (122) de l'ensemble de cathéter (104) et les languettes de retenue de pointe d'aiguille (152A, 152B) respectives ayant des première et seconde ouvertures (156A, 156B) correspondantes configurées pour permettre le passage de la canule d'aiguille (106) à travers ces dernières,
dans lequel l'ensemble d'attache de sécurité est configuré pour être dans une première position dans laquelle les premier et second bras de protection (148A, 148B) sont limités contre leur sollicitation naturelle, par le passage de la canule d'aiguille (106) à travers les première et seconde ouvertures (156A, 156B) des première et seconde languettes de retenue (152A, 152B) de sorte que les première et seconde caractéristiques de crochet (154A, 154B) s'étendant à partir des extrémités distales respectives des bras de protection (148A, 148B) sont en contact de préhension avec le raccord de cathéter (122) ; et
dans lequel l'ensemble d'attache de sécurité est configuré pour être dans une seconde position, dans laquelle la canule d'aiguille (106) est retirée, de manière proximale, à travers les première et seconde ouvertures (156A, 156B) pour permettre aux premier et second bras de protection (148A, 148B) d'être naturellement séparés par sollicitation afin de libérer le raccord de cathéter (122) du contact de préhension par les première et seconde caractéristiques de crochet (154A, 154B) et de ramener les languettes de retenue (152A, 152B) à leur position naturelle pour empêcher le mouvement distal de la pointe distale pointue (110) de la canule d'aiguille (106) dans l'attache de sécurité (132), **caractérisé par** l'attache de sécurité (132) comprenant deux languettes de retenue d'attache de sécurité (133A, 133B) opposées s'étendant à partir des côtés de la paroi proximale (146) différents de l'endroit où les premier et second bras de protection (148A, 148B) s'étendent à partir de la paroi proximale (146), les deux languettes de retenue d'attache de sécurité (133A, 133B) opposées étant configurées pour dévier lorsque l'attache de sécurité (132) est insérée dans une chambre (142) du boîtier (134) afin de permettre à l'attache de sécurité (132) d'être positionnée au moins partiellement dans le boîtier (134) et afin de fournir un ajustement avec serrage entre une ou plusieurs parmi les deux languettes de retenue d'attache de sécurité (133A, 133B) opposées et une ou plusieurs des parois définissant la chambre (142) du boîtier (134) pour empêcher le mouvement distal de l'attache de sécurité (132) hors du boîtier (134).

2. Dispositif d'insertion de cathéter selon la revendication 1, dans lequel l'attache de sécurité (132) est construite avec une fine bande de métal résilient.

3. Dispositif d'insertion de cathéter selon la revendication 1, dans lequel les première et seconde caractéristiques de crochet (154A, 154B) des premier et second bras de protection (148A, 148B), respectivement, sont formées et dimensionnées pour se conformer étroitement à un diamètre externe et à une crête d'extension et/ou à des oreilles externes du raccord de cathéter (122).

4. Dispositif d'insertion de cathéter selon la revendication 1, dans lequel les premier et second bras de protection (148A, 148B) sont couplés, de manière opérationnelle, à la paroi proximale (146).

5. Dispositif d'insertion de cathéter selon la revendication 4, dans lequel la paroi proximale (146) comprend une ouverture (150) configurée pour permettre au corps allongé de la canule d'aiguille (106) de passer à travers cette dernière, mais empêche le passage d'une caractéristique d'aiguille (114) de la canule d'aiguille (106) à travers cette dernière.

6. Dispositif d'insertion de cathéter selon la revendication 5, dans lequel, dans la seconde position, la pointe distale pointue (110) de la canule d'aiguille (106) est capturée dans l'attache de sécurité (132) étant donné que le mouvement proximal de la pointe distale pointue (110) par rapport à l'attache de sécurité (132) est empêché par interférence entre la caractéristique d'aiguille (114) et l'ouverture (150) de la paroi proximale (146) et le mouvement distal de la pointe distale pointue (110) par rapport à l'attache de sécurité (132) est empêché par les languettes de retenue (152A, 152B).

7. Dispositif d'insertion de cathéter selon la revendication 1, dans lequel les languettes de retenue d'attache de sécurité (153A, 153B) sont fabriquées avec un matériau résilient et s'étendent à partir de la paroi proximale (146) à un angle oblique.
